# EUROPEAN PATENT SPECIFICATION

(11) **EP 4 266 916 B1**
(45) Date of publication and mention of the grant of the patent: **10.06.2026**
(21) Application number: 21844323.2
(22) Date of filing: 22.12.2021
(51) Int. Cl.: A24F 40/40, A24F 40/20, A24F 40/485, A24F 40/465, A61M 11/04, A61M 15/06

(54) **AEROSOL PROVISION DEVICE**
AEROSOLBEREITSTELLUNGSVORRICHTUNG
DISPOSITIF DE FOURNITURE D'AÉROSOL

(30) Priority: 22.12.2020 GB 202020429
(43) Date of publication of application: 01.11.2023
(73) Proprietor: Nicoventures Trading Limited, London WC2R 3LA (GB)
(72) Inventor: MCGRATH, Conor, London Greater London WC2R 3LA (GB); WARREN, Luke, London Greater London WC2R 3LA (GB); BUREAU, David, London Greater London WC2R 3LA (GB); BURGESS, Jon, London Greater London WC2R 3LA (GB); THOMAS, Michael, London Greater London WC2R 3LA (GB)
(74) Representative: Dehns
(86) International application number: PCT/EP2021/087416
(87) International publication number: WO 2022/136608

(56) References cited:
- EP-A1- 3 217 817
- EP-A1- 3 664 644
- EP-A1- 3 711 568
- WO-A1-2020/025746
- WO-A1-2020/122428

## Description

### Technical Field

The present invention relates to an aerosol provision device. The present invention also relates to an aerosol provision system comprising said aerosol provision device and an article comprising aerosol generating material.

WO 2020/025746 A1 discloses an adaptor for insertion into a heating chamber. EP3711568 A1 discloses a smoking substitute device comprising a cap configured to engaged with a housing, defining an air inlet therebetween, wherein this document describes a device comprising the features mentioned in the preamble of present claim 1. EP 3664644 A1 discloses an aerosol generating device having an elastic susceptor element. EP 3217817 A1 discloses a cartridge for an electronic vapour inhaler.

### Background

Smoking articles such as cigarettes, cigars and the like burn tobacco during use to create tobacco smoke. Attempts have been made to provide alternatives to these articles that burn tobacco by creating products that release compounds without burning. Examples of such products are heating devices which release compounds by heating, but not burning, the material. The material may be for example tobacco or other non-tobacco products, which may or may not contain nicotine.

### Summary

The present invention provides an aerosol provision device comprising a heater assembly as claimed in claim 1.

In an embodiment of the above embodiment, the offset is an angular offset. The angular offset may be between 10° and 110°. Optionally the angular offset may be between 20° and 100°.

In an embodiment of any of the above embodiments, the at least part of the air passage extends substantially radially.

In an embodiment of any of the above embodiments, the air passage may extend between the heating chamber and the aperture. The aperture may be an air inlet to the flow path. The device may comprise insulation between the heater assembly and the battery. The insulation may be between the air inlet member and the battery. The insulation may comprise aerogel.

In an embodiment of any of the above embodiments, the air passage extends from a base of the heating chamber.

The heater assembly may comprise a receptacle defining the heating chamber. The receptacle may comprise the heating element. The heating element may comprise a wall of the receptacle. The receptacle may comprise a base wall. The base wall may define the base of the heating chamber.

In an embodiment of any of the above embodiments, the air inlet member defines a first bore aligned on the longitudinal axis and a second bore extending at an angle from the first bore defining the at least part of the air passage diverging from the longitudinal axis.

In an embodiment of any of the above embodiments, the heating element at least partially surrounds the heating chamber.

In an embodiment of any of the above embodiments, the heating element comprises a susceptor which is heatable by penetration with a varying magnetic field. The device may further comprise an inductor coil, wherein the inductor coil is configured to generate the varying magnetic field.

In an embodiment of any of the above embodiments, the heating element is a resistive heating element.

In accordance with some embodiments described herein, the aerosol provision device is a tobacco heating product.

In accordance with some embodiments described herein, there is provided an aerosol provision system as claimed in claim 13.

### Brief Description of the Drawings

Embodiments of the invention will now be described, by way of example only, with reference to the accompanying drawings, in which:
Figure 1 shows a front view of an aerosol provision device;
Figure 2 shows a partially exploded side view of the aerosol provision device of Figure 1 showing a chassis, end members, power source, aerosol generating assembly, replaceable article, and outer cover;
Figure 3 shows a close up schematic cross-sectional side view of part of the aerosol provision device of Figure 1;
Figure 4 shows a close up schematic cross-sectional view showing part of the aerosol generating assembly of Figure 2;
Figure 5 shows another close up schematic cross-sectional view showing a proximal part of the aerosol generating assembly of Figure 2;
Figure 6 shows another close up schematic cross-sectional view showing a distal part of the aerosol generating assembly of Figure 2;
Figure 7 shows another close up schematic cross-sectional view showing part of the aerosol generating assembly of Figure 2;
Figure 8 shows another close up schematic cross-sectional view showing part of the aerosol generating assembly of Figure 2;
Figure 9 shows a schematic cross-sectional view showing a flow path through the part of the aerosol generating assembly of Figure 3; and
Figure 10 shows a schematic cross-sectional view showing another flow path through the part of the aerosol generating assembly of Figure 3;
Figure 11 shows a close up schematic cross-sectional view showing part of the aerosol generating assembly of Figure 2;
Figure 12 shows the lip seal of Figure 11;
Figure 13 shows a cross-sectional view of a part of the lip seal of Figures 11 and 12;
Figure 14 shows a front view of an alternative aerosol provision device; and
Figure 15 shows an exploded view of the housing of the aerosol provision device of Figure 14.

### Detailed Description

As used herein, the term "aerosol generating material" includes materials that provide volatilised components upon heating, typically in the form of an aerosol. Aerosol generating material includes any tobacco-containing material and may, for example, include one or more of tobacco, tobacco derivatives, expanded tobacco, reconstituted tobacco or tobacco substitutes. Aerosol generating material also may include other, non-tobacco, products, which, depending on the product, may or may not contain nicotine. Aerosol generating material may for example be in the form of a solid, a liquid, a gel, a wax or the like. Aerosol generating material may for example also be a combination or a blend of materials. Aerosol generating material may also be known as "smokable material".

Apparatus is known that heats aerosol generating material to volatilise at least one component of the aerosol generating material, typically to form an aerosol which can be inhaled, without burning or combusting the aerosol generating material. Such apparatus is sometimes described as an "aerosol generating device", an "aerosol provision device", a "heat-not-burn device", a "tobacco heating product device" or a "tobacco heating device" or similar. In a preferred embodiment of the present invention, the aerosol-generating device of the present invention is a tobacco heating product. The non-liquid aerosol-generating material for use with a tobacco heating product comprises tobacco.

Similarly, there are also so-called e-cigarette devices, which typically vaporise an aerosol generating material in the form of a liquid, which may or may not contain nicotine. The aerosol generating material may be in the form of or be provided as part of a rod, cartridge or cassette or the like which can be inserted into the apparatus. A heater for heating and volatilising the aerosol generating material may be provided as a "permanent" part of the apparatus.

An aerosol provision device can receive an article comprising aerosol generating material for heating. An "article" in this context is a component that includes or contains in use the aerosol generating material, which is heated to volatilise the aerosol generating material, and optionally other components in use. A user may insert the article into the aerosol provision device before it is heated to produce an aerosol, which the user subsequently inhales. The article may be, for example, of a predetermined or specific size that is configured to be placed within a heating chamber of the device which is sized to receive the article.

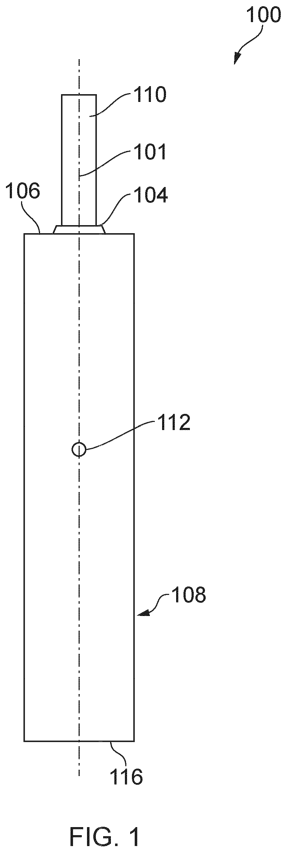

Figure 1 shows an example of an aerosol provision device 100 for generating aerosol from an aerosol generating medium/material. In broad outline, the device 100 may be used to heat a replaceable article 110 comprising the aerosol generating medium, to generate an aerosol or other inhalable medium which is inhaled by a user of the device 100.

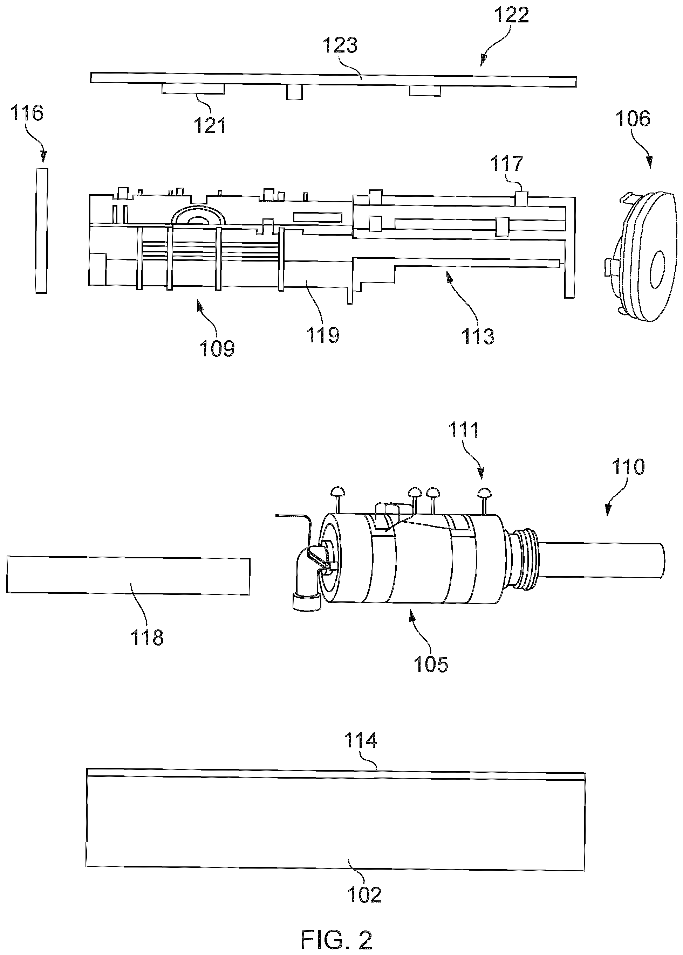

The device 100 comprises a housing 102 (including an outer cover) which surrounds and houses various components of the device 100. The device 100 has an opening 104 in one end, through which the article 110 may be inserted for heating by a heater assembly 105 (refer to Figure 2). In use, the article 110 may be fully or partially inserted into the heater assembly 105 where it may be heated by one or more components of the heater assembly 105.

The device 100 may also include a user-operable control element 112, such as a button or switch, which operates the device 100 when pressed. For example, a user may turn on the device 100 by operating the switch 112.

The device 100 defines a longitudinal axis 101.

Figure 2 depicts a schematic exploded view of the device 100 of Figure 1. The device 100 comprises the outer cover 102, a first end member 106 and a second end member 116. The device 100 includes a chassis 109, a power source 118, and an aerosol generating assembly 111 including the heater assembly 105. The device 100 further comprises at least one electronics module 122.

The outer cover 102 forms part of a device shell 108. The first end member 106 is arranged at one end of the device 100 and the second end member 116 is arranged at an opposite end of the device 100. The first and second end members 106, 116 close the outer cover 102. The first and second end members 106, 116 form part of the shell 108. The device 100 in embodiments comprises a lid (not shown) which is moveable relative to the first end member 106 to close the opening 104 when no article 110 is in place.

The device 100 may also comprise an electrical component, such as a connector/port 114, which can receive a cable to charge a battery of the device 100. For example, the connector 114 may be a charging port, such as a USB charging port. In some examples the connector 114 may be used additionally or alternatively to transfer data between the device 100 and another device, such as a computing device.

The device 100 includes the chassis 109. The chassis 109 is received by the outer cover 102. The aerosol generating assembly 111 comprises the heater assembly 105 into which, in use, the article 110 may be fully or partially inserted where it may be heated by one or more components of the heater assembly 105. The aerosol generating assembly 111 and the power source 118 are mounted on the chassis 109. The chassis 109 is a one piece component.

The chassis 109 may be formed together during manufacture, for example through an injection moulding process. Alternatively, two or more features of the chassis 109 may be separately formed initially and then formed together during a manufacturing stage to form a one-piece component, for example by a welding process.

One-piece component refers to a component of the device 100 which is not separable into two or more components following assembly of the device 100. Integrally formed relates to two or more features that are formed into a one piece component during a manufacturing stage of the component.

The first and second end members 106, 116 together at least partially define end surfaces of the device 100. For example, the bottom surface of the second end member 116 at least partially defines a bottom surface of the device 100. Edges of the outer cover 102 may also define a portion of the end surfaces. The first and second end members 116 close open ends of the outer cover 102. The second end member 116 is at one end of the chassis 109.

The end of the device 100 closest to the opening 104 may be known as the proximal end (or mouth end) of the device 100 because, in use, it is closest to the mouth of the user. In use, a user inserts an article 110 into the opening 104, operates the user control 112 to begin heating the aerosol generating material and draws on the aerosol generated in the device. This causes the aerosol to flow through the device 100 along a flow path towards the proximal end of the device 100.

The other end of the device furthest away from the opening 104 may be known as the distal end of the device 100 because, in use, it is the end furthest away from the mouth of the user. As a user draws on the aerosol generated in the device, the aerosol flows in a direction towards the proximal end of the device 100. The terms proximal and distal as applied to features of the device 100 will be described by reference to the relative positioning of such features with respect to each other in a proximal-distal direction along the axis 101.

The power source 118 is disposed at the distal end of the device 100. The chassis 109 mounts the power source 118. The chassis 109 comprises a power supply mount 119. The chassis 109 partially encloses the power source 118. The power source 118 may be, for example, a battery, such as a rechargeable battery or a non-rechargeable battery. Examples of suitable batteries include, for example, a lithium battery (such as a lithium-ion battery), a nickel battery (such as a nickel-cadmium battery), and an alkaline battery. The battery is electrically coupled to the aerosol generating assembly 111 to supply electrical power when required and under control of a controller 121 to heat the aerosol generating material. In this example, the battery is connected to the chassis 109, acting as a central support, which holds the battery 118 in place.

The power source 118 and aerosol generating assembly 111 are disposed in an axial arrangement, with the power source 118 at the distal end of the device 100 and the aerosol generating assembly 111 at the proximal end of the device 100. Other configurations are anticipated. The chassis 109 comprises an aerosol generating assembly mount 113.

The device 100 further comprises at least one electronics module 122. The electronics module 122 may comprise, for example, a printed circuit board (PCB) 123. The PCB 123 may support at least one controller 121, such as a processor, and memory. The PCB 123 may also comprise one or more electrical tracks to electrically connect together various electronic components of the device 100. For example, the battery terminals may be electrically connected to the PCB 123 so that power can be distributed throughout the device 100. The connector 114 may also be electrically coupled to the battery 118 via the electrical tracks. The chassis 109 comprises a PCB mount 117.

The aerosol generating assembly 111 is an inductive heating assembly and comprises various components to heat the aerosol generating material of the article 110 via an inductive heating process. Induction heating is a process of heating an electrically conducting object (such as a susceptor) by electromagnetic induction. An induction heating assembly may comprise an inductive element, for example, one or more inductor coils, and a device for passing a varying electric current, such as an alternating electric current, through the inductive element. The varying electric current in the inductive element produces a varying magnetic field. The varying magnetic field penetrates a susceptor suitably positioned with respect to the inductive element, and generates eddy currents inside the susceptor. The susceptor has electrical resistance to the eddy currents, and hence the flow of the eddy currents against this resistance causes the susceptor to be heated by Joule heating. In cases where the susceptor comprises ferromagnetic material such as iron, nickel or cobalt, heat may also be generated by magnetic hysteresis losses in the susceptor, i.e. by the varying orientation of magnetic dipoles in the magnetic material as a result of their alignment with the varying magnetic field. In inductive heating, as compared to heating by conduction for example, heat is generated inside the susceptor, allowing for rapid heating. Further, there need not be any physical contact between the inductive heater and the susceptor, allowing for enhanced freedom in construction and application. Whilst an inductive heating means is described, any suitable heating means may be used, such as a resistive heating means.

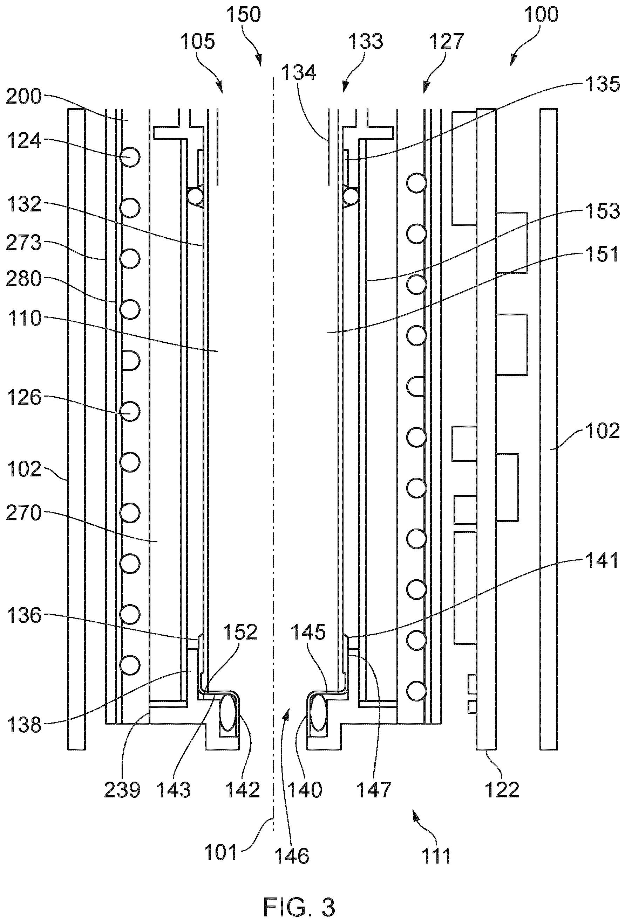

Figure 3 shows a close up side view of part of the device 100 in partial cross-section. The outer cover 102 encloses the aerosol generating assembly 111. The aerosol generating assembly 111 of the device 100 comprises the heater assembly 105 and an inductor coil assembly 127. The inductor coil assembly 127 extends around the heater assembly 105. The inductor coil assembly 127 includes a first inductor coil 124 and a second inductor coil 126. The inductor coil assembly 127 also comprises a coil support 200.

The heater assembly 105 includes a susceptor arrangement 132 (herein referred to as "a susceptor"). The susceptor 132 of this example is hollow. For example, the article 110 can be inserted into the susceptor 132. In this example the susceptor 132 is tubular, with a circular cross section. The susceptor 132 defines a first portion of the heater assembly 105. The susceptor 132 has a generally constant diameter along its axial length. The susceptor 132 has a flared portion 134 at a first, proximal, end 133. The flared portion 134 diverges outwardly. The flared portion 134 defines an outwardly extending lip 135. That is the lip 135 has a greater diameter than the outer diameter of the main portion of the susceptor 132. The lip 135 acts to minimise contact of the susceptor 132 with other components at the first end 133. This arrangement helps with a low heat transmission, for example through conduction, when the susceptor 132 is heated.

The susceptor 132 is formed from an electrically conducting material suitable for heating by electromagnetic induction. The susceptor in the present example is formed from a carbon steel. It will be understood that other suitable materials may be used, for example a ferromagnetic material such as iron, nickel or cobalt.

The heater assembly 105 also comprises a funnel part 140. The funnel part 140 is at a second, distal, end 136 of the susceptor 132. The funnel part 140 protrudes from the susceptor 132. In embodiments, the susceptor 132 and funnel part 140 are a one-piece component.

The susceptor 132 and the funnel part 140 define a receptacle 150. The receptacle 150 defines a heating chamber 151 within which aerosol generating material is received. For example, the article 110 can be inserted into the receptacle 150. Figure 3 shows a portion of the article 110 received within the heating chamber 151. The article 110 of this example comprises aerosol generating material. The aerosol generating material is positioned within the susceptor 132. The article 110 may also comprise other components such as a filter, wrapping materials and/or a cooling structure.

In this example, the receptacle is substantially tubular, with a circular cross section. The receptacle 150 is open at one end. The receptacle 150 has a base wall 152 and a peripheral wall 153. The base wall 152 defines a base of the heating chamber 151. The receptacle 150 in the present arrangement is formed by the susceptor 132 and the funnel part 140. In embodiments, the receptacle 150 is separably formed from the susceptor 132 and funnel part 140. That is, the receptacle 150 is a different component. For example, the susceptor may be an internal susceptor protruding in the heating chamber 151. The receptacle 150 may be formed by a body. The receptacle may be a cup. The funnel part 140 in embodiments is omitted.

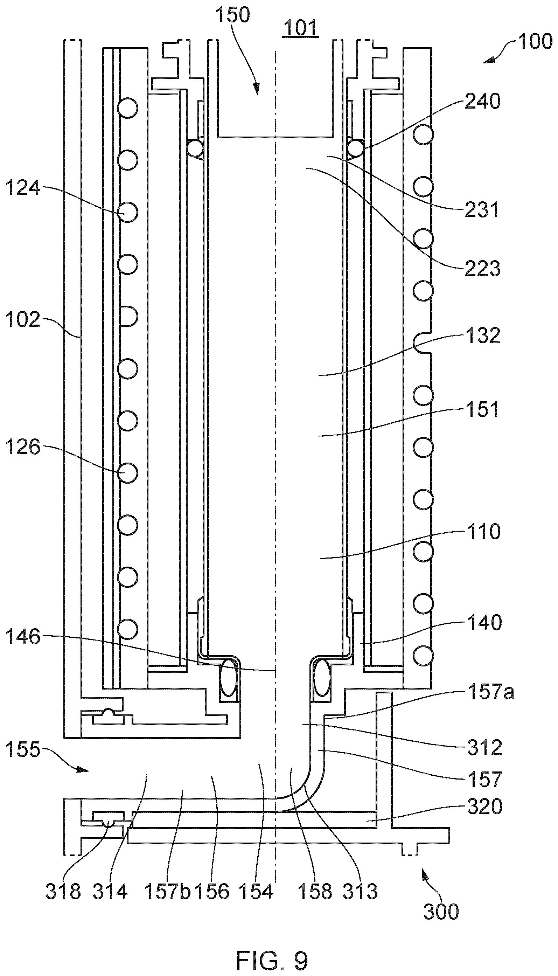

A flow path 154 extends through the device 100 as more clearly shown in Figure 9. The flow path 154 is defined from an aperture 155 in the housing 102 through the heating chamber 151 to the opening 104. When an article 110 is at least partially received in the heating chamber 151, the flow path extends through the article 110, and so may extend to a distal end of the article 110. An air passage 156 is defined between the aperture 155 and the heating chamber 151, as will be described in detail below with reference to Figure 9. The aperture 155 acts as an air inlet.

The funnel part 140 has a thimble arrangement. The funnel part 140 is at the second, distal, end 136 of the susceptor 132. The funnel part 140 defines a second portion of the heater assembly 105. The funnel part 140 comprises a first section 141 having a first diameter and a second section 142 having a second diameter. An intermediate section 143 extends between the first and second sections 141, 142. The intermediate section 143 in the present embodiment defines the base wall 152 of the receptacle 150. The first section 141 is tubular and extends in the axial direction. The second section 142 is tubular and extends in the axial direction. The funnel part 140 form an air channel 146 that defines part of the air passage 156 from the heating chamber 151 to the aperture 155. The intermediate section 143 forms a shoulder 145. The shoulder 145 acts as a stop to limit insertion of the article 110 into the receptacle. The shoulder 145 extends on a substantially perpendicular plane to a longitudinal axis of the receptacle.

The first section 141 has an inner diameter which is greater than the inner diameter of the second section 142. The funnel part 140 therefore converges from the first section 141 to the second section 142.

The first section 141 and the susceptor 132 partially overlap with each other at one end of the susceptor 132. In an example, the overlap is between about 1mm and about 3mm. In this particular example, the overlap is 2mm. In examples, there is no overlap. In such an example the susceptor 132 and funnel part 140 abut. The first section 141 overlaps the second, distal, end 136 of the susceptor 132. The first section 141 is generally cylindrical and has an inner diameter substantially corresponding to the outer diameter of the susceptor 132. The first section 141 abuts the susceptor 132. A juncture 147 is formed between the first section 141 of the funnel part 140 and the susceptor 132. The juncture 147 assists with forming a thermally conductive path between the susceptor 132 and the funnel part 140.

The juncture 147 is a fluidly sealed juncture. A fluid seal is formed between the susceptor 132 and the funnel part 140. As such a fluidly sealed fluid path is defined between the opposing ends of the susceptor 132 and the funnel part 140. The heating chamber 151 defined by the susceptor 132 therefore forms a fluid seal air path with the air channel 146 formed by the funnel part 140, said fluid seal air path forming a part of the air path through the device 100 with the air passage 156.

The fluid seal at the juncture 147 is formed in embodiments by a mechanical fabricated joint, for example a weld. The fluid seal at the juncture 147 is formed by a laser weld process, however it will be understood that other methods may be used such as brazing and adhering. The funnel part 140 is formed from a thermally conductive material. In embodiments, the funnel part 140 is formed from a carbon steel. The funnel part in embodiments is formed from the same material as the susceptor 132. The juncture is configured to retain a fluid seal when the susceptor 132 is at its predetermined operating temperature. By such processes the susceptor 132 and funnel part 140 are fabricated as a one-piece component.

The sealed fluid path between the susceptor 132 and the funnel part 140 therefore extends through the heater assembly 105 from one open end of the heater assembly 105 to the other open end of the heater assembly 105. As such, any fluid flow through the heater assembly 105 is contained in the heater assembly 105. A dry zone may be defined outside the heater assembly 105.

The abutment of the susceptor 132 and the funnel part 140 provides for heat transfer by conduction from the susceptor 132 to the funnel part 140. As such, it is possible to aid passive heating of the funnel part 140. By passively heating the funnel part 140 it is possible to restrict the rate of build up of condensate in the device 100.

The funnel part 140 is axially spaced from the inductor coil assembly 127. In particular, the second section 142 of the funnel part 140 is axially spaced from the inductor coil assembly 127. As such, there is minimal or no direct heating of the funnel part 140 by the inductor coil assembly 127. The funnel part 140 may lie adjacent to the inductor coil assembly 127 in an axial direction.

An air inlet arrangement 300 defines the air passage 156 between the aperture 155 and the heating chamber 151. An air inlet member 157 extends between the funnel part 140 and the housing 102 as shown in Figure 9. The air inlet member 157 comprises a bore 158. The air inlet member 157 communicates with the funnel part 140 such that the air channel 146 of the funnel part 140 fluidly communicates with the bore 158 of the air inlet member 158. The air inlet arrangement 300 will be described in detail below.

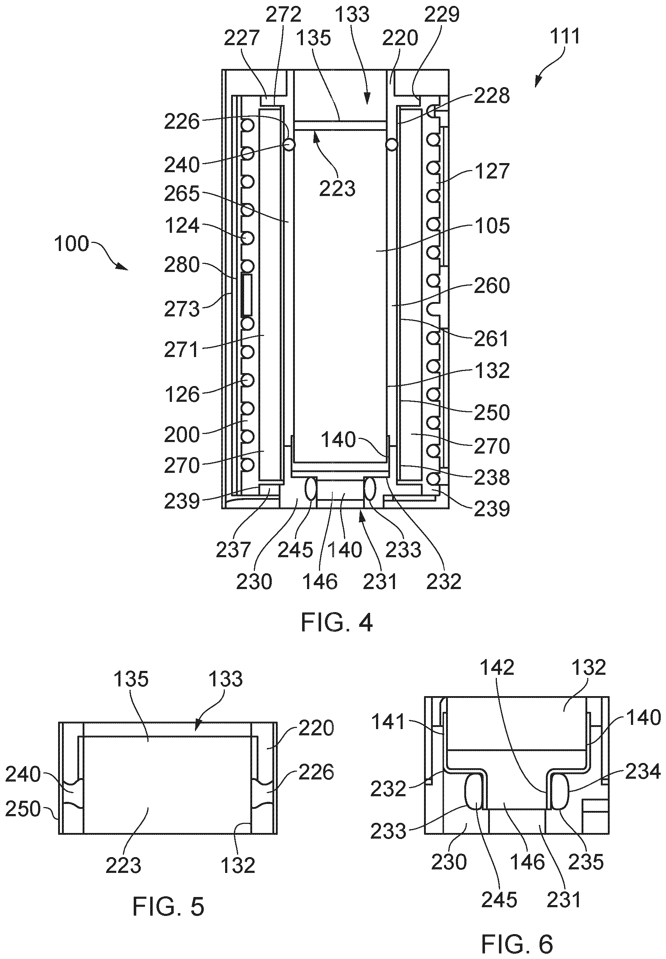

Referring in particular to Figures 4 to 8, the device 100 comprises a first end support 220 and a second end support 230. The air inlet member 157 in the present arrangement is formed with the second end support 230. In embodiments, the air inlet member 158 and second end support are separate elements.

The heater assembly 105 extends between the first and second end supports 230. A barrier member 250 extends between the first end support 220 and the second end support 230. The barrier member 250 acts as a support member.

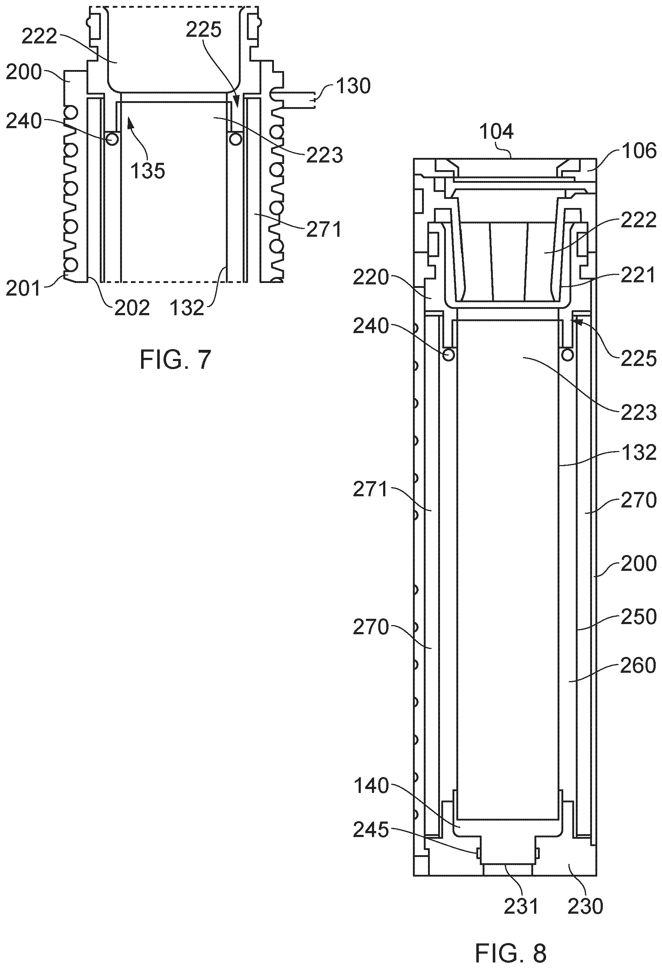

The first end support 220 engages the first, proximal, end of the heater assembly 105 to hold the susceptor 132 in place. The first end support 220 acts as an expansion chamber, as described below. Referring to Figures 7 and 8 in particular, the first end support 220 extends away from the first end of the susceptor 132 towards the opening 104 of the device. Located at least partially within the first end support 220 is a retention arrangement 221, such as a retention clip, to abut and hold the article 110 when received within the device 100. The first end support 220 is connected to the end member 106.

The first end support 220 comprises a chamber 222. The chamber 222 is configured to receive the article 110 therethrough. The retention arrangement 221 is in the chamber 222. The chamber 222 has an inner diameter greater than the diameter of the article 110. The first end support 220 forms a first, proximal, collar for the heater assembly 105. A bore 223 extends therethrough. As shown, for example, in Figures 7 and 8, a distally facing shoulder 225 is defined on the inner surface of the bore 223. The distally facing shoulder 225 locates with the lip 135 of the susceptor 132 when the susceptor is received by the first end support 220.

Referring now in particular to Figures 4 and 5, the first end support 220 forms a sealing rim 226 on a distal side of the first end support 220. The distal sealing rim 226 extends about the bore 223. A first mounting flange 227 extends from a first, proximal, end outer surface 228 of the first end support 220. The first mounting flange 227 extends circumferentially and is spaced from the sealing rim 226. The first mounting flange 227 upstands from the first end outer surface 228 and forms a first, proximal, end mounting surface 229. The first, proximal, end outer surface 228 and the first end mounting surface 229 define a stepped configuration. The first end mounting surface 229 has a greater diameter than the first end outer surface 228. In embodiments, the first end outer surface 228 and first end mounting surface define first and second step faces.

Referring in particular to Figures 4 to 8, the device 100 further comprises the second end support 230 which engages the funnel part 140 at the second, distal, end of the susceptor 132 to hold the heater assembly 105 in place. The second end support 230 forms a second, distal, collar for the heater assembly 105. In embodiments in which the funnel part is omitted, the second end support 230 engages the susceptor 132 directly. The second end support 230 extends away from the second end of the susceptor 132 towards the distal end of the device 100.

Referring in particular to Figures 4 and 6, the second end support 230 acting as the air inlet member 157 forms part of the air passage 156 between the heating chamber 151 and the aperture 155. The second end support 230 is configured to at least partially receive the funnel part 140. The inner surface of the second end support 230 is stepped. The inner surface comprises a first stepped region 232 with a first step, and a second stepped region 233 with a second step. The first stepped region 232 receives the first section 141 of the funnel part 140. The second stepped region 233 receives the second section 142 of the funnel part 140. The second stepped region 233 includes a first sealing face 234. The second stepped region 233 includes a second sealing face 235. The first sealing face 234 is an internal circumferentially extending face. The second sealing face 235 is a circumferentially extending face extending on a plane substantially perpendicular to the longitudinal axis 101.

A second mounting flange 237 extends from a second, distal, outer surface 238 of the second end support 230. The second mounting flange 237 extends circumferentially and is spaced from a proximal end of the second end support 230. The second mounting flange 237 upstands from the second end outer surface 238 and forms a second, distal, end mounting surface 239. The second, distal, end outer surface 238 and the second, distal, end mounting surface 239 define a stepped configuration. The second end mounting surface 239 has a greater diameter than the second end outer surface 238. In embodiments, the second end outer surface 238 and second end mounting surface 239 define first and second step faces.

The barrier member 250 extends between the first end support 220 and the second end support 230. The barrier member 250 extends between the first and second end supports 220, 230. The barrier member 250 together with the first and second end supports 220, 230 encloses the heater assembly 105. This acts to assist with thermally isolating the heater assembly 105 from other components of the device 100. The barrier member 250 is a hollow, tubular member.

In embodiments, the barrier member 250 is formed from a non-metallic material to assist with limiting interference with magnetic induction. In this particular example, the barrier member 250 is constructed from polyether ether ketone (PEEK). The first and second end supports 220, 230 are constructed from PEEK. Other suitable materials are possible. Parts formed from such materials help ensure that the barrier member 250 remains rigid/solid when the susceptor is heated. The heater assembly 105, the barrier member 250, and the first and second end supports 220, 230 are coaxial around the central longitudinal axis of the susceptor 132. The barrier member 250 may help insulate the various components of the device 100 from the heat generated in the susceptor 132.

Referring now in particular to Figures 4 to 6, a first sealing member 240 forms a fluid seal between the heating assembly 105 and the first end support 220. The first sealing member 240 is a circumferentially extending member. The first sealing member 240 comprises a silicon rubber seal. Other suitable materials may be used. The first sealing member 240 is resilient. The material is configured to be stable when the heating assembly 105 is at operating temperature. A second sealing member 245 forms a fluid seal between the heating assembly 105 and the second end support 230. The second sealing member 245 is a circumferentially extending member. The second sealing member 245 comprises a silicon rubber seal. Other suitable materials may be used. The second sealing member 245 is resilient. The material is configured to be stable when the heating assembly 105 is at operating temperature.

In embodiments, the first sealing member 240 is on the first end support 220 and seals with the heater assembly 105. In embodiments, the second sealing member 245 is on the second end support 230 and seals with the heater assembly 105. The second sealing member 245 is on the second section 142 of the funnel part 140. In embodiments, the second sealing member 245 is on the first section 141 of the funnel part 140. In such an embodiment, the second sealing member 245 seals against a proximal rim of the second end support 230.

The first sealing member 240 and second sealing member 245 form a sealed air flow path through the second sealing member 250, heater assembly 105 and first sealing member 240.

A fluidly sealed cavity 260 is formed between the heater assembly 105 and the barrier member 105. The fluidly sealed cavity 260 forms an insulation chamber. The cavity 260 provides an air gap. A fluidly sealed enclosure 261 is formed around part of the heater assembly 105. The fluidly sealed enclosure is formed by the barrier member, first and second sealing members 240, 245, heater assembly 105 and the second end support 230. In some embodiments, the first end support 220 forms part of the enclosure 261. In some embodiments, the fluidly sealed enclosure 261 is formed by the barrier member, heater assembly 105 and first and second sealing members 240, 245. In embodiments, the gap between the heater assembly 105 and the barrier member 105 is between about 0.8mm and 1mm. In embodiments, the gap is about 0.9mm.

A sensor, such as a thermocouple 265, is disposed in the fluidly sealed cavity 260. The thermocouple 265 is mounted on the susceptor 132. The thermocouple 265 is configured to determine the temperature of the susceptor 132. The thermocouple 265 directly detects the temperature of the susceptor 132. The device 100 may comprise two or more thermocouples 132 configured to determine the temperature of the susceptor 132. The provision of the fluidly sealed cavity 260 helps to isolate the thermocouples 265 from atmosphere external to the fluidly sealed cavity 260. The provision of the fluidly sealed cavity 260 helps to isolate the thermocouples 265 from the air flow path through the device 100. As such, condensate from the air flow path is restricted from flowing to the thermocouples 265.

Referring now to Figure 9, the air inlet arrangement 300 will now be described in detail. The aperture 155 is formed through the housing 102 between an outer side and an inner side. The aperture 155 communicates the air inlet arrangement 300 with external to the device 100. The air inlet arrangement 300 comprises the air inlet member 157. The air inlet member 157 defines the air passage 156 extending between the heating chamber 151 and the aperture 155 in the housing 102. The air inlet member 157 communicates between the funnel part 140 and the housing 102. The air inlet member 157 is tubular. In embodiments, the air inlet member 157 and the funnel part 140 are formed as a one piece component.

A sealed fluid air path extends from the aperture 155, through the air passage 156 of the air inlet member 230, through the air channel 146 of the funnel part 140, into the heating chamber 151, and through an article 110 inserted in the heating chamber. In use, when the user inhales, air will enter the aperture 155, and travel through the air inlet member 230 and the heating chamber 151, and the aerosol generating material of the article 110. In embodiments in which the funnel part 140 is not present, the air passage 156 extends directly from the heating chamber 231 to the aperture 155.

The device 100 defines a longitudinal axis 101. The longitudinal axis 101 extends in the longitudinal direction of the elongate housing 102.

The heater assembly 105 has a longitudinal axis. In the present embodiment, the longitudinal axis of the heater assembly 105 is coaxial with the longitudinal axis of the device 100. However, in embodiments, the axes are offset. The longitudinal axis of the heater assembly is defined by a longitudinal axis of the susceptor and coil.

The heating chamber 151 defines a longitudinal axis. The heating chamber 151 defines an insertion axis for the article 110. The longitudinal axis of the heating chamber 151 is defined by the receptacle 150. In embodiments, the axes of the heating chamber 151 and the heater assembly 105 are coaxial. In embodiments, the axes are offset.

The air inlet member 157 is offset from the longitudinal axis of the heating chamber 151. That is, at least part of the air inlet member 157 extends away from the longitudinal axis. The air inlet member 157 as shown in Figure 9 comprises a first member portion 157a and a second member portion 157b. The first member portion 157a extends from the heater assembly 105. The second member portion 157b extends from the first member portion 157a to the housing 102.

The air passage 156 is offset from the longitudinal axis of the heating chamber 151. That is, at least part of the air passage 156 is non-coaxial with the longitudinal axis of the heating chamber 151. The air passage 156 diverges from the longitudinal axis. The air passage 156 comprises a first passage portion 312 aligned with the longitudinal axis of the heating chamber 151. The first passage portion 312 extends through the first member portion 157a and is coaxial with the longitudinal axis of the heating chamber 151. A second passage portion 314 of the air passage 156 extends through the second member portion 157b and is offset from the first passage portion 312. The second passage portion 314 of the air passage 156 extends at an angle to the first passage portion. A bend 313 is defined between the first and second passage portions 312, 314. The air passage 156 has an axis at an angular offset to the longitudinal axis of the heating chamber 151.

The first passage portion 312 may be omitted. In some embodiments, the first passage portion 312 is also offset from the longitudinal axis.

The second passage portion 314 extends in a radial direction, i.e. perpendicular to the longitudinal axis of the heating chamber 105. The first passage portion 312 communicates with the receptacle 150. The first passage portion 312 communicates with the funnel part 140, or in embodiments in which the funnel part is omitted, the susceptor 132. The second passage portion 314 extends to the aperture 155 in the housing 102.

In embodiments, the air passage 156 is substantially linear. The bore defining such an air passage 156 is offset from the longitudinal axis of the heating chamber 105. The air passage 156 may follow any suitable path having at least a portion offset from the longitudinal axis.

The air passage 156 has a substantially constant cross-section, and/or a substantially constant cross sectional area. For example, in the present embodiment the air passage 156 has a substantially circular cross section with a substantially constant diameter. In embodiments, the second section 142 of the funnel part 140 has substantially the same diameter. Thus, the air passage 156 has a substantially uniform cross section and/or cross sectional area from the aperture 155 to the heating chamber 231. The aperture 155 is aligned with the second passage portion 314, and may have a similar cross sectional shape and size thereas. The air inlet member 230 defines a tubular arrangement. The tubular arrangement may have a cross-section other than circular. The tubular member may be formed integrally with the air inlet member. Aspects of the above arrangement aid with providing a smooth air flow.

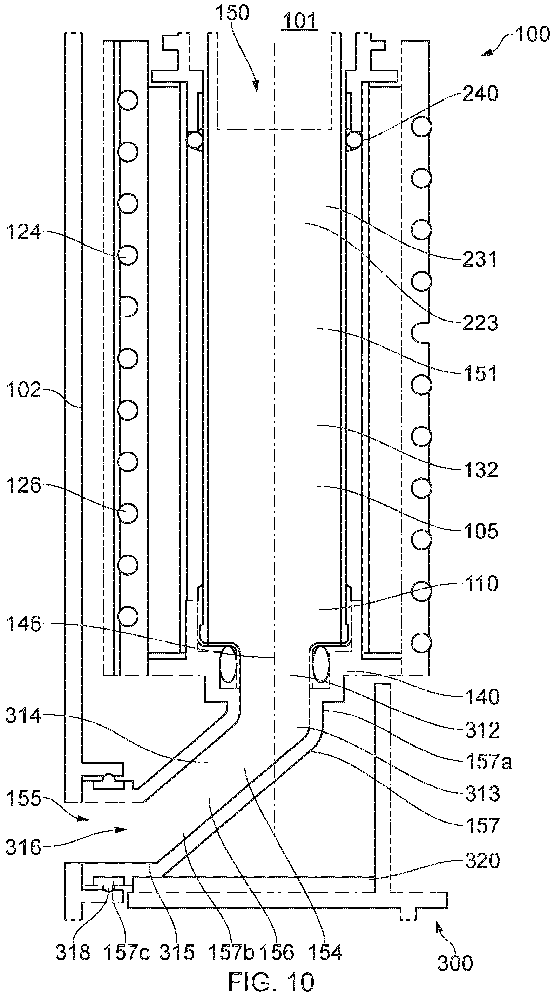

Figure 10 shows another air inlet arrangement 300. The device of figure 10 generally corresponds to the device 100 described above with respect to Figure 9, and so a detailed description is omitted herein. In this configuration, the air inlet member 157 comprises a section extending at an acute angle to the longitudinal axis. That is, at least part of the air passage extends non-parallel and non-perpendicular to the longitudinal axis. The air inlet member 157 communicates with the side wall of the housing 102. The bend 313 defined between the first and second passage portions 312, 313 is non-right angled, such that the second passage portion 314 extends in non-perpendicular direction to the longitudinal axis of the heating chamber 105. The second passage portion 314 extends at an angle of 50 degrees to the longitudinal axis of the heating chamber 105. The air passage 156 thus bends by 50 degrees, whilst the air inlet member 157 has a bend of 130 degrees therein. The air inlet member 157 also comprises a third member portion 157c, which extends from the second member portion 157b to the housing 102, and is offset from the second member portion 157b. The air passage 156 further comprises a third passage portion 316, which extends at an angle to the second passage portion 314. A bend 315 is defined between the second and third passage portions 312, 314. The third passage portion may extend in a radial direction, i.e. perpendicular to the longitudinal axis 101. The third passage portion that extends to the aperture 155 in the housing. The bend 315 may correspond to the bend 313 between the first and second passage portion 312, 314, such that the combination of the two bends is 90 degrees. In embodiments, the first and/or third passage portions 157a,c may be omitted. In these embodiments, the second passage portion 157b may extend to the funnel portion 140 and/or the aperture 155 in the housing.

Providing such an angled air passage may provide improved air flow into the device to an article 110 located in the heating chamber of the device by providing a better distribution of air across the base of the article, and a more laminar flow. A non-radially extending angled air passage may aid in providing a better distributed air flow, i.e. which does not all flow through the centre of an article 110 inserted in the device in use. This provides a sensory benefit to a user.

An bend or angle in the air inlet member 231 (e.g. where there is a 90 degree bend in the air passage, as in embodiment of Figure 9) may aid in providing a more compact device as it allows for a better arrangement for components such as the battery in the device 100 by providing a suitable air inlet in a smaller space.

The air inlet member 231 is located between the heating chamber and the battery. This separation of the heating chamber and the battery, and/or the provision of a "cool" air flow between the heating chamber and the battery protects the battery from the temperature of the heating chamber (i.e allows the battery to remain at a cooler temperature). However, the components may be arranged in a different manner in some embodiments.

The skilled person will understand that a range of angular offsets of the air passage of the air inlet member may provide at least some of the above described benefits. The angular offset may be between 10 and 110 degrees, such as between 20 and 100 degrees, such as 50 degrees. The air inlet member may have a bend of between 60 and 170 degrees, such as between 110 and 160 degrees, such as 130 degrees, therein.

There is a layer of insulation 320 provided between the air inlet chamber and the battery, such as between the air inlet member and the battery. This layer of insulation may be formed from any suitable material, such as an aerogel. As discussed above, the arrangement of the components may provide better thermal distribution within the device 100. Additionally or alternatively, the arrangement of the components may be beneficial for the weight distribution within the device. However, the layer of insulation may be omitted in some embodiments.

The air inlet member may be formed from any suitable material, including thermoplastics such as PEEK (polyether ether ketone). PEEK is able to withstand the operating temperature of the device without substantially deforming.

The air inlet member may be sealed to the housing 102 using any suitable means. In the embodiments depicted in Figures 9 and 10, o-ring seals 318 are used. These may be made from any suitable material, such as epoxy resin.

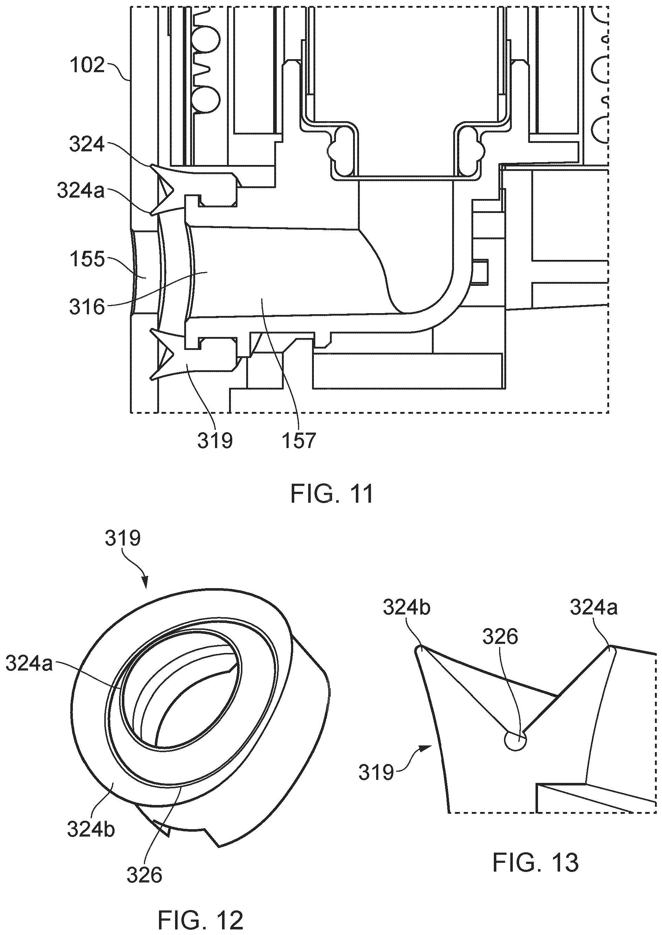

Figure 11 shows a configuration for sealing the air inlet member 157 to the housing 102. The sealing configuration comprises a lip seal 319. The lip seal 319 may be formed from any suitable material, such as silicon rubber. The lip seal 319 is a radial lip seal. The lip seal fluidly seals the air inlet member 157 with the housing 102.

The lip seal 319 is mounted on the air inlet member 157. The lip seal 319 extends between the air inlet member 157 and the housing 102. The lip seal surrounds the aperture 155 in the housing 102. The lip seal 319 encircles the aperture 155 on the inner side of the housing 102. The aperture 155 acts as an air inlet. The lip seal 319 abuts the outer cover of the housing 102. The outer cover forms a side wall of the housing 102 and extends in a longitudinal direction.

The lip seal 319 may be separately formed before being attached or fitted to the air inlet member 157. As shown in Figure 11, the lip seal extends beyond an end of the air inlet member in a longitudinal direction of the distal end of the air inlet member 157. The distal end is the end distal from the heating chamber. The lip seal 319 is overmoulded on the end of the air inlet member 157. The lip seal may be mounted on the end of the air inlet member 157 by other fixing means, such as a fixture, push fitting, and bonding. As can be seen in Figures 11 and 12, the end of the lip seal distal to the air inlet member 157 comprises two longitudinally extending sealing lips 324a, 324b. Each lip may form a radial ring. The inner lip 324a tends radially inwardly. The outer lip 324b tends radially outwardly. This may aid sealing. In embodiments, a single lip or more than two lips may be provided. When the aerosol generating device is assembled, the heater assembly 105 is slid into the outer cover of the housing 102 with the air inlet member 157. The lip seal 319 contacts the inner side of the outer cover and slides along the surface. The lip seal 319 is positioned over the inner side of the aperture 155. The lip seal encircles the rim of the aperture 155. The lip seal 319 may deform to be compressed between the air inlet member 157 and the housing 102 (compression not shown). This forms a seal between the air inlet member 157 and the outer cover of the housing 102, and helps provide a tolerance for the assembly of the device. The flanges may reduce stress and concentrations of stress on the lip seal 319. A channel 326 between the flanges, as can be seen in Figure 13, may be included to further reduce stress concentrations on the lip seal 319.

In embodiments, the aperture is a single opening. In embodiments, the aperture 155 comprises the housing 102 comprises an air permeable region defining the aperture 155. The aperture 155 acts as an air inlet.

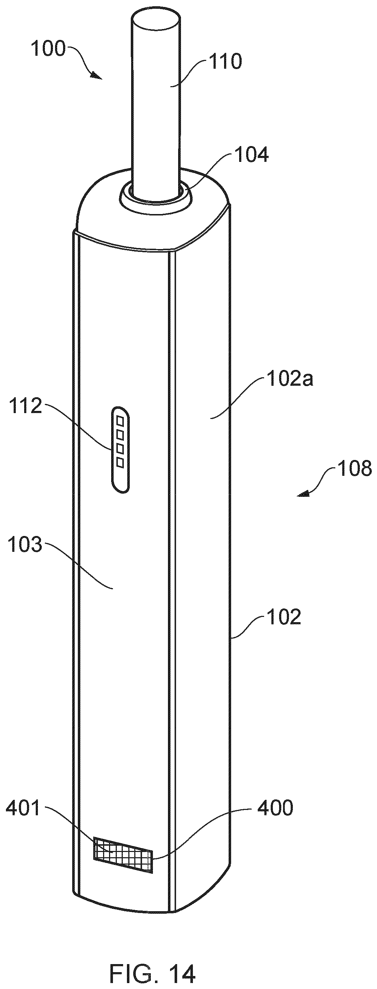

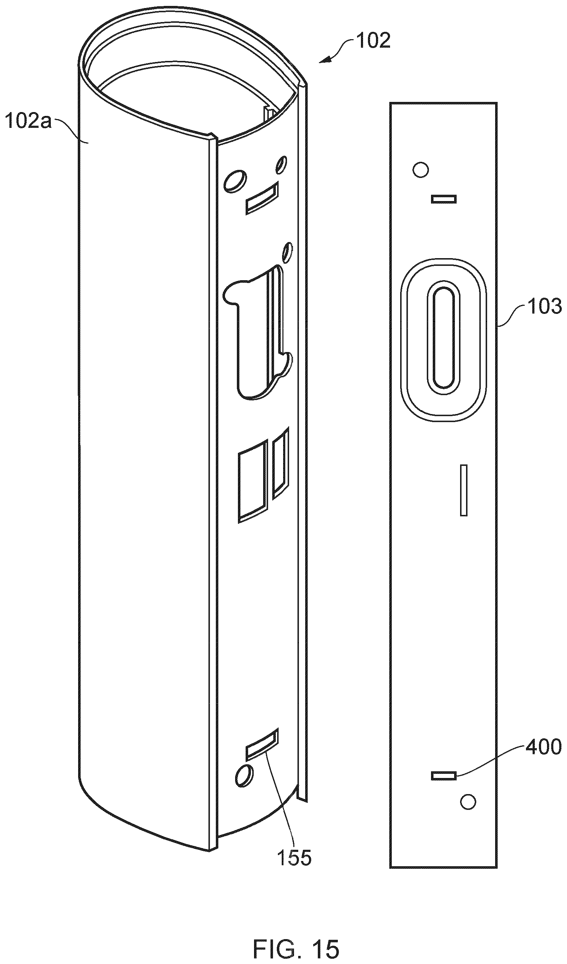

Referring now to Figures 14 and 15, an aerosol provision device 100 similar to that of Figure 1 is shown. The housing 102 comprises a main body 102a and a removable facia 103. The removable facia 103 acts as a separable portion. The removable facia 103 is removable from the main body 102a. The removable facia 103 covers a portion of the main body 102a. The removable facia 103 in the present embodiment forms part of the outer cover. By providing the removable facia 103, this allows for customisation of the device. The removable facia 103 may be removable by the user. When attached to the housing, the removable facia 103 may overlap a panel of the main body 102a, i.e. such that when the removable facia 103 is removed, the interior of the device is not exposed.

The separable portion 103 is arranged to be mounted on the main body 102a. Figure 15 shows an exploded view of the main body 102a and removable facia 103 of Figure 14 in a separated condition. The main body 102a may comprise an indented portion arrange to receive the removable facia. The removable facia 103 may be attached to the housing by any suitable means, such as a snap fit or magnets. The separable portion may be a panel.

The removable facia 103 may include an air permeable region 400. The air permeable region 400 acts as an air inlet. The air permeable region is arranged to align with the aperture 155 in the main body 102a. The air permeable region 400 is arranged to cover the aperture 155 in the housing through which air is drawn to the air flow passage.

The air permeable region 400 may comprise a mesh 401. The mesh forms part of the separable portion 103. The mesh 401 extends across the aperture 155 when the removable facia 103 is mounted with the main body 102a of the housing 102.

The mesh may be formed from any suitable material, such as a metal or a polymer. The metal may be stainless steel, aluminium, nickel, gold, silver, palladium, or platinum. The mesh may be comprised of mixture of materials, such as the an alloy of any of the above described metals. Alternatively, the mesh could be plated, for example nickel plated steel.

The mesh may be acid etched or laser cut from a metal foil. Alternatively, the mesh may be formed from a polymer. The mesh includes a plurality of mesh holes. The mesh holes may be any suitable shape, such as rectangular, square, circular, or another non-asymmetric shape.

In embodiments, different air permeable arrangements are used. For example, the air permeable region may comprise an array of vent holes formed through the panel. The vent holes may be slits,, circular, rectangular, or any other suitable shape, such as a suitable non-asymmetric shape. Each vent hole may have a flow area of less than 2mm², optionally less than 1.5mm² 1mm², optionally less than 0.5mm², and optionally less than 0.1mm². For example, each vent hole may have a flow area of 0.5mm² to 1.5mm², such as about 1mm².

Alternatively, the air permeable region 400 may be an opening arranged to align with the aperture 155 in the housing.

When the removable facia is removed, this may allow access to the aperture 105, such as for cleaning.

The air permeable region 400 prevents debris or detritus from entering the air passage, for example when the device is stored in a user's bag.

The air permeable region 400 is removable from the aperture by removing the removable facia 103. This allows the air passage to be cleaned by a user, such as if condensation forms. The removable facia 103 may be releasably attached by any suitable means, such as mechanical means such as clips, or magnetic means.

The removable facia 103 may be releasably attachable to the housing by any suitable means such as mechanical means such as clips, or magnetic means. The means may be located on the removable portion and/or the main portion. For example, the removable portion and the main portion may comprise corresponding clips or magnets.

The above embodiments are to be understood as illustrative examples of the invention. Further embodiments of the invention are envisaged. It is to be understood that any feature described in relation to any one embodiment may be used alone, or in combination with other features described, and may also be used in combination with one or more features of any other of the embodiments, or any combination of any other of the embodiments.

## Claims

1. An aerosol provision device (100) comprising:
a housing (102);
a battery (118);
a heater assembly (105) having:
a heating chamber (151) defining a longitudinal axis (101) arranged to receive at least a portion of an article (110) comprising aerosol generating material; and
a heating element configured to heat aerosol generating material received in the heating chamber (151);
**characterised by** the device (100) having an air inlet member (157) comprising a tubular member and forming an air passage (156) at one end of the heating chamber (151), the air passage (156) defining part of a flow path (154) with the heating chamber (151);
wherein at least part of the air passage (156) defined by the air inlet member (157) is offset from the longitudinal axis (101) of the heating chamber (151);
wherein the air passage (156) extends to an aperture (155) in the housing (102); and
wherein the air passage (156) communicates with the housing (102) between the heater assembly (105) and the battery (118).

2. The aerosol provision device (100) of claim 1, wherein the offset is an angular offset; optionally wherein the angular offset is between 10° and 110°, optionally wherein the angular offset is between 20° and 100°.

3. The aerosol provision device (100) of claim 1, wherein the at least part of the air passage (156) extends substantially radially.

4. The aerosol provision device (100) of any preceding claim , wherein the air passage (156) extends between the heating chamber (151) and the aperture (155).

5. The aerosol provision device (100) of any preceding claim, wherein the aperture (155) is an air inlet to the flow path (156).

6. The aerosol provision device (100) of any preceding claim, comprising insulation (320) between the heater assembly (105) and the battery (118); optionally wherein the insulation (320) is between the air inlet member (157) and the battery (118); optionally wherein the insulation comprises aerogel.

7. The aerosol provision device (100) of any of claims 1-6, wherein the air passage (156) extends from a base of the heating chamber (151).

8. The aerosol provision device (100) of any of claims 1-7, wherein the air inlet member (157) defines a first bore aligned on the longitudinal axis (101) and a second bore extending at an angle from the first bore defining the at least part of the air passage (156) diverging from the longitudinal axis (101).

9. The aerosol provision device (100) of any of claims 1-8, wherein the heating element at least partially surrounds the heating chamber (151).

10. The aerosol provision device (100) of any of claims 1-9, wherein the heating element comprises a susceptor (132) which is heatable by penetration with a varying magnetic field; optionally further comprising an inductor coil (127), wherein the inductor coil (127) is configured to generate the varying magnetic field.

11. The aerosol provision device (100) of any of claims 1-10, wherein the heating element is a resistive heating element.

12. The aerosol provision device of any of claims 1-11, wherein the aerosol provision device (100) is a tobacco heating product.

13. An aerosol provision system comprising:
an aerosol provision device (100) according to any of claims 1-12; and
an article (110) dimensioned to be at least partially received within the heater assembly (105);
wherein the article comprises aerosol generating material, for example a a non-liquid aerosol generating material.

## Patentansprüche

1. Aerosolbereitstellungsvorrichtung (100), umfassend:
ein Gehäuse (102);
eine Batterie (118);
eine Heizungsbaugruppe (105), die Folgendes aufweist:
eine Heizkammer (151), die eine Längsachse (101) definiert und so angeordnet ist, dass sie mindestens einen Abschnitt eines Artikels (110) aufnimmt, der aerosolerzeugendes Material umfasst; und
ein Heizelement, das so konfiguriert ist, dass in der Heizkammer (151) aufgenommenes aerosolerzeugendes Material erhitzt wird;
**dadurch gekennzeichnet, dass** die Vorrichtung (100) ein Lufteinlasselement (157) aufweist, das ein rohrförmiges Element umfasst und einen Luftdurchgang (156) an einem Ende der Heizkammer (151) bildet, wobei der Luftdurchgang (156) einen Teil eines Strömungswegs (154) mit der Heizkammer (151) definiert;
wobei mindestens ein Teil des von dem Lufteinlasselement (157) definierten Luftdurchgangs (156) von der Längsachse (101) der Heizkammer (151) versetzt ist;
wobei sich der Luftdurchgang (156) zu einer Öffnung (155) in dem Gehäuse (102) erstreckt; und
wobei der Luftdurchgang (156) mit dem Gehäuse (102) zwischen der Heizungsbaugruppe (105) und der Batterie (118) verbunden ist.

2. Aerosolbereitstellungsvorrichtung (100) nach Anspruch 1, wobei der Versatz ein Winkelversatz ist; optional, wobei der Winkelversatz zwischen 10° und 110° liegt; optional, wobei der Winkelversatz zwischen 20° und 100° liegt.

3. Aerosolbereitstellungsvorrichtung (100) nach Anspruch 1, wobei sich mindestens ein Teil des Luftdurchgangs (156) im Wesentlichen radial erstreckt.

4. Aerosolbereitstellungsvorrichtung (100) nach einem der vorstehenden Ansprüche, wobei sich der Luftdurchgang (156) zwischen der Heizkammer (151) und der Öffnung (155) erstreckt.

5. Aerosolbereitstellungsvorrichtung (100) nach einem vorstehenden Anspruch, wobei die Öffnung (155) ein Lufteinlass für den Strömungsweg (156) ist.

6. Aerosolbereitstellungsvorrichtung (100) nach einem der vorstehenden Ansprüche, umfassend eine Isolierung (320) zwischen der Heizungsbaugruppe (105) und der Batterie (118); optional, wobei sich die Isolierung (320) zwischen dem Lufteinlasselement (157) und der Batterie (118) befindet; optional, wobei die Isolierung Aerogel umfasst.

7. Aerosolbereitstellungsvorrichtung (100) nach einem der Ansprüche 1 bis 6, wobei sich der Luftdurchgang (156) von einem Sockel der Heizkammer (151) erstreckt.

8. Aerosolbereitstellungsvorrichtung (100) nach einem der Ansprüche 1 bis 7, wobei das Lufteinlasselement (157) eine erste Bohrung definiert, die auf der Längsachse (101) ausgerichtet ist, und eine zweite Bohrung, die sich in einem Winkel von der ersten Bohrung erstreckt und mindestens einen Teil des Luftdurchgangs (156) definiert, der von der Längsachse (101) abweicht.

9. Aerosolbereitstellungsvorrichtung (100) nach einem der Ansprüche 1 bis 8, wobei das Heizelement die Heizkammer (151) mindestens teilweise umgibt.

10. Aerosolbereitstellungsvorrichtung (100) nach einem der Ansprüche 1 bis 9, wobei das Heizelement einen Suszeptor (132) umfasst, der durch Durchdringen mit einem variierenden Magnetfeld erwärmt werden kann; optional weiter umfassend eine Induktionsspule (127), wobei die Induktionsspule (127) so konfiguriert ist, dass sie das variierende Magnetfeld erzeugt.

11. Aerosolbereitstellungsvorrichtung (100) nach einem der Ansprüche 1 bis 10, wobei das Heizelement ein ohmsches Heizelement ist.

12. Aerosolbereitstellungsvorrichtung nach einem der Ansprüche 1 bis 11, wobei die Aerosolbereitstellungsvorrichtung (100) ein Tabakerhitzungsprodukt ist.

13. Aerosolbereitstellungssystem, umfassend:
eine Aerosolbereitstellungsvorrichtung (100) nach einem der Ansprüche 1 bis 12; und
einen Artikel (110), der so dimensioniert ist, dass er mindestens teilweise in die Heizungsbaugruppe (105) aufgenommen werden kann;
wobei der Artikel aerosolerzeugendes Material umfasst, beispielsweise ein nicht flüssiges aerosolerzeugendes Material.

## Revendications

1. Dispositif (100) de fourniture d'aérosol comprenant :
un boîtier (102) ;
une batterie (118) ;
un ensemble de chauffage (105) présentant :
une chambre de chauffage (151) définissant un axe longitudinal (101) agencée pour recevoir au moins une partie d'un article (110) comprenant un matériau générateur d'aérosol ; et
un élément chauffant conçu pour chauffer un matériau générateur d'aérosol reçu dans la chambre de chauffage (151) ;
**caractérisé en ce que** le dispositif (100) présente un organe d'entrée d'air (157) comprenant un organe tubulaire et formant un passage d'air (156) à une extrémité de la chambre de chauffage (151), le passage d'air (156) définissant une partie d'un trajet d'écoulement (154) avec la chambre de chauffage (151) ;
dans lequel au moins une partie du passage d'air (156) défini par l'organe d'entrée d'air (157) est décalée par rapport à l'axe longitudinal (101) de la chambre de chauffage (151) ;
dans lequel le passage d'air (156) s'étend jusqu'à une ouverture (155) dans le boîtier (102) ; et
dans lequel le passage d'air (156) communique avec le boîtier (102) entre l'ensemble de chauffage (105) et la batterie (118).

2. Dispositif (100) de fourniture d'aérosol selon la revendication 1, dans lequel le décalage est un décalage angulaire ; éventuellement, dans lequel le décalage angulaire est compris entre 10° et 110°, éventuellement, dans lequel le décalage angulaire est compris entre 20° et 100°.

3. Dispositif (100) de fourniture d'aérosol selon la revendication 1, dans lequel au moins une partie du passage d'air (156) s'étend sensiblement radialement.

4. Dispositif (100) de fourniture d'aérosol selon une revendication précédente, dans lequel le passage d'air (156) s'étend entre la chambre de chauffage (151) et l'ouverture (155).

5. Dispositif (100) de fourniture d'aérosol selon une quelconque revendication précédente, dans lequel l'ouverture (155) est une entrée d'air vers le trajet d'écoulement (156).

6. Dispositif (100) de fourniture d'aérosol selon une quelconque revendication précédente, comprenant une isolation (320) entre l'ensemble de chauffage (105) et la batterie (118) ; éventuellement dans lequel l'isolation (320) est située entre l'organe d'entrée d'air (157) et la batterie (118) ; éventuellement dans lequel l'isolation comprend un aérogel.

7. Dispositif (100) de fourniture d'aérosol selon l'une quelconque des revendications 1 à 6, dans lequel le passage d'air (156) s'étend à partir d'une base de la chambre de chauffage (151).

8. Dispositif (100) de fourniture d'aérosol selon l'une quelconque des revendications 1 à 7, dans lequel l'organe d'entrée d'air (157) définit un premier alésage aligné sur l'axe longitudinal (101) et un deuxième alésage s'étendant sous un angle par rapport au premier alésage, définissant l'au moins une partie du passage d'air (156) divergent de l'axe longitudinal (101).

9. Dispositif (100) de fourniture d'aérosol selon l'une quelconque des revendications 1 à 8, dans lequel l'élément chauffant entoure au moins partiellement la chambre de chauffage (151).

10. Dispositif (100) de fourniture d'aérosol selon l'une quelconque des revendications 1 à 9, dans lequel l'élément chauffant comprend un suscepteur (132) qui peut être chauffé par pénétration avec un champ magnétique variable ; comprenant éventuellement en outre une bobine d'inducteur (127), dans lequel la bobine d'inducteur (127) est configurée pour générer le champ magnétique variable.

11. Dispositif (100) de fourniture d'aérosol selon l'une quelconque des revendications 1 à 10, dans lequel l'élément chauffant est un élément chauffant résistif.

12. Dispositif de fourniture d'aérosol selon l'une quelconque des revendications 1 à 11, dans lequel le dispositif (100) de fourniture d'aérosol est un produit de chauffage de tabac.

13. Système de fourniture d'aérosol, comprenant :
un dispositif (100) de fourniture d'aérosol selon l'une quelconque des revendications 1 à 12 ; et
un article (110) dimensionné pour être reçu au moins partiellement à l'intérieur de l'ensemble de chauffage (105) ;
dans lequel l'article comprend un matériau générateur d'aérosol, par exemple un matériau générateur d'aérosol non liquide.
